# EUROPÄISCHE PATENTSCHRIFT

(11) **EP 0 702 943 B1**
(45) Veröffentlichungstag und Bekanntmachung des Hinweises auf die Patenterteilung: **09.06.1999**
(21) Anmeldenummer: 95112835.4
(22) Anmeldetag: 16.08.1995
(51) Int. Cl.: A61F 5/01, A61F 5/14

(54) **Fussorthese**
Foot orthesis
Orthèse à pied

(30) Priorität: 27.08.1994 DE 4430493
(43) Veröffentlichungstag der Anmeldung: 27.03.1996
(73) Patentinhaber: Otto Bock Orthopädische Industrie Besitz- und Verwaltungs-Kommanditgesellschaft, 37115 Duderstadt (DE)
(72) Erfinder: Baise, Monique, Dr., D-83229 Aschau (DE)
(74) Vertreter: Gramm, Werner, Prof. Dipl.-Ing.

(56) Entgegenhaltungen:
- EP-A- 0 269 946
- EP-A- 0 468 351
- WO-A-94/05236
- US-A- 4 753 228

## Beschreibung

Die Erfindung betrifft eine Fußorthese mit folgenden Merkmalen:
- eine geschlossene, lediglich im Fersenbereich (4) seitlich geöffnete und hier mit einem Schließmechanismus versehene Kunststoffschale (2) in Form einer verwindungsstabilen Ringorthese, geeignet, eine externe Versteifung im Sprunggelenk zu bilden;
- die Kunststoffschale (2) weist einen etwas hochgezogenen, steif ausgebildeten Stützbereich (6) auf, geeignet, sich am Außenfuß am distalen Ende der Fibula (7) abzustützen;
- die Kunststoffschale (2) ist geeignet, das Talo-Navikular- und Calcaneo-Cuboid-Gelenk nach proximal zu überspannen und extern zu arthrodesieren;

Der Erfindung liegen die nachfolgenden Überlegungen und Erkenntnisse zugrunde:

Bei der Entstehung des reversiblen spastischen Knickplattfußes liegt ein Entwicklungsdefizit vor. Aufgrund der späten Vertikalisation verbleibt der Fuß bei Spastikern in der Supinationsstellung des Säuglingsalters. Die Vorfußrotation ist ausgeblieben. Durch die Koordinationsstörung fällt die Funktion der intrinsischen Muskulatur der Fußsohle aus. Die Sohle ist plan und reliefarm. Die spastischen Verspannungen der langen Muskeln wie z. B. der Achillessehne, der Zehenextensoren und der Fibularis-Gruppe überwiegen. Der Tibialis anterior hat einen zu weit distal gelegenen Ansatz. Das Fußlängsgewölbe wird nicht unterstützt. Die Sehne des Tibialis posterior luxiert nach vorn auf den Knöchel. Die Folge dieses mangelhaften Zusammenspiels der Muskulatur ist der Zusammenbruch des Fußgewölbes in Höhe des Chopart-Gelenkes. Durch den Zug der Achillessehne gerät der Calcaneus in eine Valgusabweichung und rotiert unter den Talus. Dieser wiederum hat die Abstützung in der Höhe des Sustentaculum Tali verloren und kippt nach unten. Das untere Sprunggelenk orientiert sich neu. Die Rotationsachse ist nicht mehr schräg, sondern horizontal. Die talo-navikularen und calcaneo-cuboidalen Gelenke werden parallel. Es erfolgt eine zunehmend passive Mobilität der plantaren Sohle. Aufgrund des Rotationsdefizits im Vorfuß folgt dieser der Valgusabweichung des Rückfußes in einer immer vorhandenen Abduktionsfehlstellung. Die spastische Verspannung der langen Zehenflexoren wandert in der Pronationshaltung nach lateral und zieht die Zehen in die Abduktionsfehlstellung.

Die eingangs beschriebene Fußorthese läßt sich der EP 0 269 946 A2 entnehmen. Es handelt sich hier um eine für das Sprunggelenk von Patienten vorgesehene Stützeinrichtung mit einem, aus steifem, dünnem Material bestehenden U-förmigen Bügel, der den Fuß des Patienten untergreift, wobei die Bügelschenkel seitlich an der Wade anliegend bis über den Knöchelbereich nach oben stehen, und mit einem Verbindungsmittel, das die vordere Öffnung des Bügels oberhalb des Ristes und die hintere Einstiegöffnung oberhalb der Ferse eng umschließt. Dabei weist der Bügel eine mit den Schenkeln einstückige, oberhalb der Ristöffnung beginnende starre Vorderwand sowie an den oberen Enden der Schenkel nach hinten anschließende und mit diesen einstückige steife Lappen auf, deren Enden einander im Bereich der Achillessehne wenigstens nahezu berühren. Das Verbindungsmittel besteht aus einem 2-teiligen Verschluß, dessen Teile außen an den Schenkeln und/oder den Lappen befestigt sind.

Der Erfindung liegt die Aufgabe zugrunde, für einen spastischen Knickplattfuß oder Knickplattfuß anderer Genese eine geeignete Orthese zu entwickeln.

Diese Aufgabe wird gemäß der Erfindung dadurch gelöst, daß die Kunststoffschale auch geeignet ist, eine externe Versteifung im Chopart-Gelenk zu bilden, daß der genannte Stützbereich der Kunststoffschale lappenförmig und besonders steif ausgebildet ist, daß der proximale Rand der Kunststoffschale beim Innenfuß unterhalb des Innenknöchels liegt, und daß die Kunststoffschale im plantaren Bereich des Fersenbeines einen Ausschnitt aufweist.

Durch die externe Versteifung im Chopart-Gelenk und im unteren Sprunggelenk (T-Arthrodesenprinzip) wird eine Restauration des physiologischen gotischen Bogens unter Belastung im Fußlängsgewölbe bei gleichzeitiger Verbesserung der Sohleninsuffizienz erreicht. Dabei wird der funktionellen Aufrichtung des Rückfußes größte Bedeutung beigemessen. Über den seitlichen Schließmechanismus im Fersenbereich ergibt sich eine Zugwirkung auf den vorderen Ring der Orthese im Bereich des Chopart-Gelenkes. Beim Schließen des Fersenteils entsteht somit eine dynamische Korrekturwirkung.

Die erfindungsgemäße Orthese kann als TR-Ringorthese bezeichnet werden, wobei TR steht für Talus-Repositionsorthese.

Durch den erfindungsgemäß gestalteten Stützbereich um den Außenknöchel herum entsteht beim Gehen ein Drehmoment, wobei sich die aufrichtende Komponente stabilisiert.

Je mehr die Kunststoffschale am Fußrücken das Talo-Navikulargelenk nach proximal überspannt und arthrodesiert, desto günstiger ist das funktionelle Ergebnis der Versorgung. Das gleiche gilt für die Höhe der dorsalen Fersenklammer. Distal überspannt die Kunststoffschale das Chopart'sche Gelenk vorzugsweise um ca. 1 cm.

Der plantare Bereich des Fersenbeines ist im Belastungsbereich längsoval in A-P-Richtung ausgeschnitten, um einen ungehinderten Bodenkontakt zu ermöglichen und eine weitere steilere Stellung des Calcaneus unter Belastung zu erreichen. Es wird somit eine Rotation (Aufrichtung) des Rückfußes (Varisierung) erreicht.

Das Korrekturprinzip läßt sich wie folgt beschreiben:

Mit der TR-Ringorthese wird eine Korrektur des Rückfußes durch eine Rotationsbewegung erreicht. Da der Talus nach unten gesenkt ist, ist beim Repositionsvorgang zunächst eine Spitzfußstellung des Rückfußes erforderlich. In der Spitzfußstellung läßt sich der Calcaneus unter den Talus bewegen. Der Calcaneus kann sein anatomisches Verhältnis zum Talus wiederfinden. Der luxierte Talus wird bei der Dorsalextension des Fußes unter Mitnahme des Calcaneus angehoben und reponiert. Ein plantarer Druck in Höhe des Talo-Navikulargelenkes ist nicht notwendig. Dieser Druck ist sogar unerwünscht, da sich die Spastizität durch eine Belastung der plantaren Sohle verstärken würde. Das erreichte Ergebnis wird durch eine externe, ringförmige Arthrodese über dem Chopartgelenk und dem unteren Sprunggelenk gehalten.

Die Orthese ist erfindungsgemäß so gestaltet, daß die Korrekturkräfte von ringförmig angelegten Formteilen aufgenommen werden, wobei eine hohe Steifigkeit der Orthese im Bereich dieser Korrekturkräfte erreicht wird. Hingegen kann die Kunststoffschale im Fersenbereich semirigide bis rigide ausgebildet sein, um das Anlegen der Orthese zu erleichtern.

Die Orthese kann erfindungsgemäß als Schalenkonstruktion in Spritzgießtechnik hergestellt werden, wobei als Material vorzugsweise ein faserverstärktes Thermoplast Verwendung findet.

Die Ränder der Kunststoffschale sind vorzugsweise wulstförmig verdickt und nach außen abgerundet, um einerseits ein Nacharbeiten in diesen kritischen Zonen zu ermöglichen und andererseits Kantendruck zu vermeiden.

Wesentlich für die Erzielung guter Ergebnisse ist eine hohe Formstabilität der Kunststoffschale, die in erster Linie durch deren geschlossene Konstruktion erreicht wird.

Es ist vorteilhaft, wenn zur variablen Einstellung der Fersenweite der Schließmechanismus ein Klettverschluß mit Rückrollschlaufe ist.

In einer alternativen Ausführungsform kann aber auch ein den Fersenbereich hintergreifendes, im Querschnitt angenähert U-förmig ausgebildetes Schalenteil vorgesehen werden, das gegenüber der Kunststoffschale in A-P-Richtung verstellbar und in der gewünschten Position an der Kunststoffschale beispielsweise über einen Rastenverschluß fixierbar ist.

Für beide vorstehend beschriebenen Alternativlösungen gilt, daß die Kunststoffschale nach dem Anlegen auch im Fersenbereich geschlossen und starr ausgebildet ist, wobei über den Schließmechanismus eine Zugwirkung auf den vorderen Ring der Orthese ausgeübt wird.

Die erfindungsgemäße Ringorthese umfaßt einen separat von der Kunststoffschale ausgebildeten, innenliegenden Weichwandliner, der den Fuß manschettenförmig umschließt. Dieser Weichwandliner, der federnde Eigenschaften aufweisen, ein geschlossener Silikonmantel sein sollte und eine Dicke von etwa 4 mm haben kann, wird zuerst über den Fuß gezogen, bevor die Kunststoffschale angezogen wird. Zur Erleichterung des Anziehens kann der Weichwandliner im Fußrückenbereich einen Einsteigeschlitz aufweisen. In diesem Bereich kann durch eine dünne Lasche ein unangenehmer Kantendruck verhindert werden.

Durch zusätzliche, fest mit der Orthese verbundene Polsterungen läßt sich die Wirkung der durch den Weichwandliner gebildeten Polstermanschette noch verbessern.

Durch einen lateral in dem für die Überspannung des Fußrückens bestimmten Bereich der Kunststoffschale angebrachten Fibulakeil läßt sich eine zusätzliche Korrekturkraft erzielen.

Das Anziehen der Orthese ist anfangs nicht einfach und bedarf der Übung. Wichtig ist hierbei eine korrigierende Rotation des Rückfußes und des Vorfußes, wobei eine leichte Spitzfußstellung den Widerstand reduziert.

In der Zeichnung ist eine als Beispiel dienende Ausführungsform der Erfindung dargestellt. Es zeigen:
- **Figur 1** -: in stark schematisierter Darstellung eine Fußorthese in Seitenansicht;
- **Figur 2** -: die Darstellung gemäß Figur 1 in Vorderansicht;
- **Figur 3** -: die Darstellung gemäß Figur 1 in Rückansicht;
- **Figur 4** -: in gegenüber Figur 1 vergrößertem Maßstab in Seitenansicht den Außenfuß mit einer angelegten Ringorthese;
- **Figur 5** -: den Innenfuß der Darstellung gemäß Figur 4 und

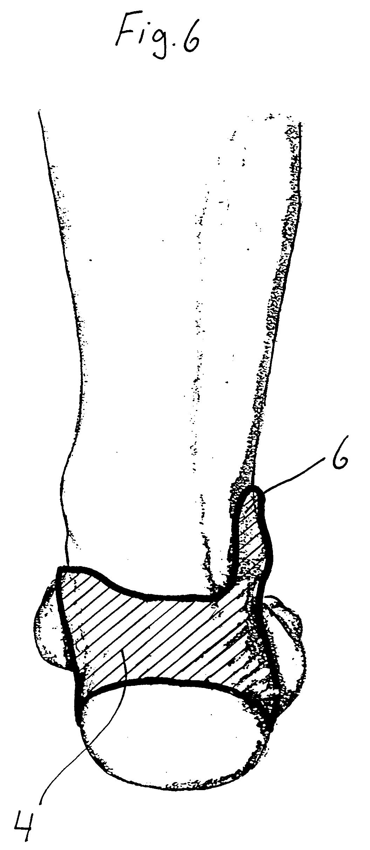

- **Figur 6** -: die Rückansicht der Darstellung gemäß Figur 4.

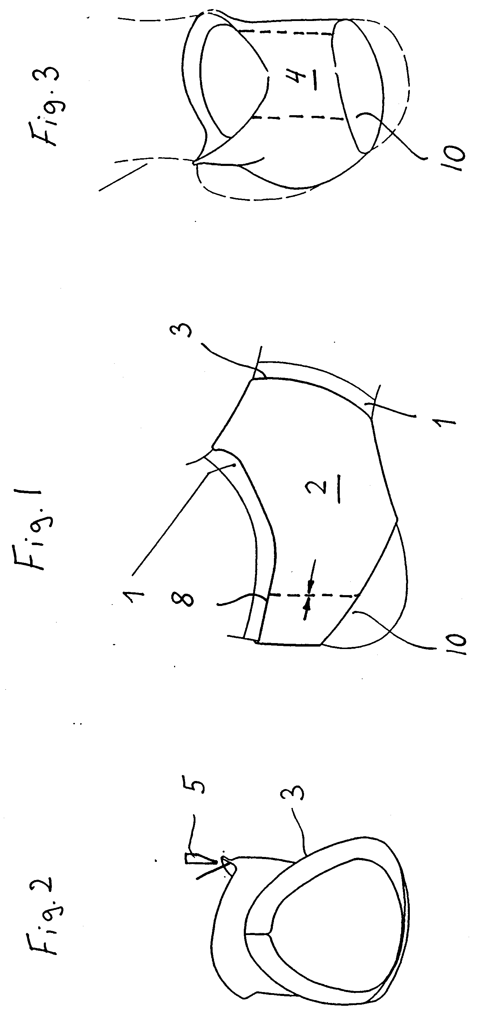

Figur 1 zeigt einen Innenfuß. Der Fuß ist von einem Weichwandliner 1 umschlossen, über den eine Kunststoffschale 2 gestreift ist. Diese Kunststoffschale 2 ist geschlossen ausgebildet und lediglich im Fersenbereich 4 seitlich öffenbar und bildet eine verwindungsstabile Ringorthese, die zur externen Versteifung im Chopartgelenk und im unteren Sprunggelenk dient. Der vordere Rand 3 der Kunststoffschale 2 ist rundum geschlossen ausgebildet, während die Kunststoffschale 2 den Fersenbereich 4 zangenförmig umgreift, der seitlich geöffnet und hier mit einem in der Zeichnung nicht näher dargestellten Schließmechanismus verschließbar ist.

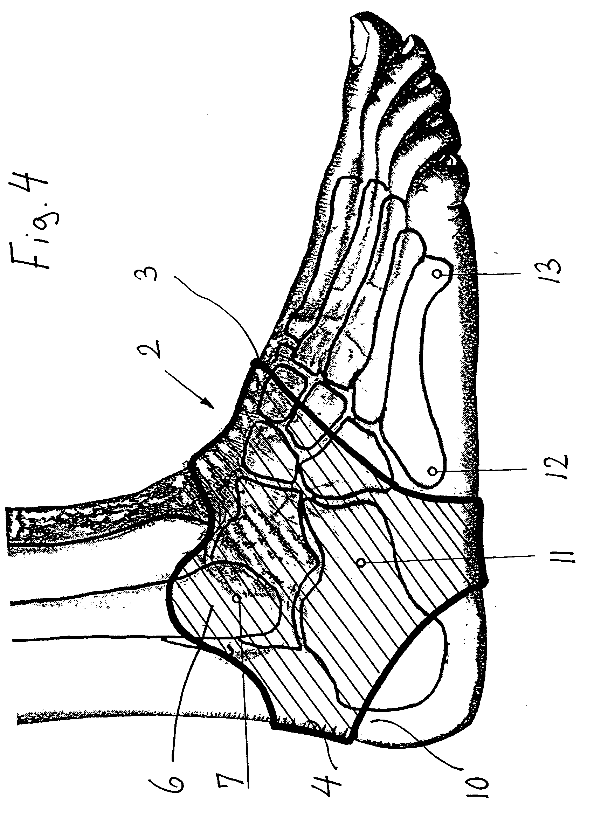

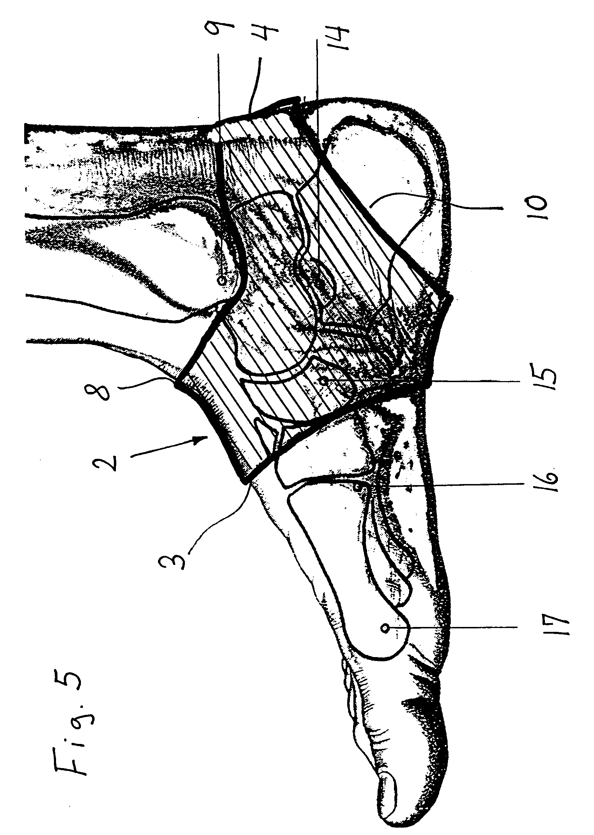

Insbesondere Figur 4 läßt erkennen, daß sich die Kunststoffschale 2 mit einem etwas hochgezogenen, lappenförmigen und besonders steif ausgebildeten Stützbereich 6 am Außenknöchel 7 abstützt. Am Fußrücken überspannt die Kunststoffschale 2 das Talo-Navikular- und Calcaneo-Gelenk um etwa 1 cm nach proximal und arthrodesiert extern. Gemäß Figur 5 liegt beim Innenfuß der proximale Rand 8 der Kunststoffschale 2 unterhalb des Innenknöchels 9. Im plantaren Bereich des Fersenbeines weist die Kunststoffschale 2 einen Ausschnitt 10 auf.

Gemäß den Figuren 4 und 5 überdeckt die Kunststoffschale 2 im Fußaußenbereich den Processus trochlearis 11, läßt aber die Basis 12 des Metatarsus V frei, dessen Köpfchen mit dem Bezugszeichen 13 gekennzeichnet ist. Im Innenfuß deckt die Kunststoffschale 2 die Talus-Stütze 14 (Sustentaculum Tali) sowie den Knochenhöcker des Kahnbeines 15 (Tuberositas des os naviculare) ab, während die Basis 16 des Metatarsus I freibleibt, dessen Köpfchen mit dem Bezugszeichen 17 versehen ist.

Der Weichwandliner 1 ist zur Verbesserung der Übersichtlichkeit ausschließlich in Figur 1 angedeutet.

## Patentansprüche

1. Fußorthese mit folgenden Merkmalen:
- eine geschlossene, lediglich im Fersenbereich (4) seitlich geöffnete und hier mit einem Schließmechanismus versehene Kunststoffschale (2) in Form einer verwindungsstabilen Ringorthese, geeignet, eine externe Versteifung im Sprunggelenk zu bilden;
- die Kunststoffschale (2) weist einen etwas hochgezogenen, steif ausgebildeten Stützbereich (6) auf, geeignet, sich am Außenfuß am distalen Ende der Fibula (7) abzustützen;
- die Kunststoffschale (2) ist geeignet, das Talo-Navikular- und Calcaneo-Cuboid-Gelenk nach proximal zu überspannen und extern zu arthrodesieren;
**dadurch gekennzeichnet,** daß die Kunststoffschale (2) auch geeignet ist, eine externe Versteifung im Chopart-Gelenk zu bilden,
daß der genannte Stützbereich (6) der Kunststoffschale (2) lappenförmig und besonders steif ausgebildet ist,
daß der proximale Rand (8) der Kunststoffschale (2) beim Innenfuß unterhalb des Innenknöchels (9) liegt, und
daß die Kunststoffschale (2) im plantaren Bereich des Fersenbeines einen Ausschnitt (10) aufweist.

2. Fußorthese nach Anspruch 1, **dadurch gekennzeichnet**, daß die Kunststoffschale (2) geeignet ist, das Chopart'sche Gelenk distal etwa 1 cm zu überspannen.

3. Fußorthese nach Anspruch 1 oder 2, **gekennzeichnet durch** eine in Spritzgußtechnik hergestellte Kunststoffschale (2).

4. Fußorthese nach Anspruch 3, **dadurch gekennzeichnet**, daß die Kunststoffschale (2) aus faserverstärktem Thermoplast besteht.

5. Fußorthese nach einem der vorhergehenden Ansprüche, **dadurch gekennzeichnet**, daß die Ränder der Kunststoffschale (2) wulstförmig verdickt und nach außen abgerundet sind.

6. Fußorthese nach einem der vorhergehenden Ansprüche, **dadurch gekennzeichnet**, daß die Kunststoffschale (2) im Fersenbereich (4) zumindest semirigide ausgebildet ist.

7. Fußorthese nach einem der vorhergehenden Ansprüche, **dadurch gekennzeichnet**, daß zur variablen Einstellung der Fersenweite der Schließmechanismus ein Klettverschluß mit Rückrollschlaufe ist.

8. Fußorthese nach einem der Ansprüche 1 bis 6, **gekennzeichnet durch** ein den Fersenbereich (4) hintergreifendes, im Querschnitt angenähert U-förmig ausgebildetes Schalenteil, das gegenüber der Kunststoffschale (2) in A-P-Richtung verstellbar und in der gewünschten Position an der Kunststoffschale (2) beispielsweise über einen Rastenverschluß fixierbar ist.

9. Fußorthese nach einem der vorhergehenden Ansprüche, **dadurch gekennzeichnet**, daß die Kunststoffschale (2) mit einem separaten, innenliegenden Weichwandliner (1) ausgestattet ist, geeignet, den Fuß manschettenförmig zu umschließen.

10. Fußorthese nach Anspruch 9, **dadurch gekennzeichnet**, daß der Weichwandliner (1) im Fußrückenbereich einen Einsteigeschlitz aufweist.

11. Fußorthese nach Anspruch 9 oder 10, **dadurch gekennzeichnet**, daß der Weichwandliner (1) federnde Eigenschaften aufweist und ein geschlossener Silikonmantel ist.

12. Fußorthese nach einem der vorhergehenden Ansprüche, **gekennzeichnet durch** einen lateral in dem für die Überspannung des Fußrückens bestimmten Bereich der Kunststoffschale (2) angebrachten Fibulakeil (5).

## Claims

1. Foot orthesis with the following features:
- a closed plastics material shell (2) in the form of a torsionally rigid ring orthesis, which is only open at the side in the heel region (4) and is provided in this case with a closing mechanism, designed to form an external reinforcement in the ankle joint;
- the plastics material shell (2) comprises a somewhat raised, rigidly constructed support region (6), designed to be supported against the outer foot at the distal end of the fibula (7);
- the plastics material shell (2) is designed to bridge and externally arthrodise the talonavicular and calcaneo-cuboid joint towards the proximal end;
characterised in that the plastics material shell (2) is also designed to form an external reinforcement in the Chopart's joint, the above-mentioned support region (6) of the plastics material shell (2) is flap-shaped and particularly rigid, the proximal edge (8) of the plastics material shell (2) lies beneath the inner ankle (9) in the case of the inner foot and in the plantar region of the heel bone the plastics material shell (2) comprises a cut-away section (10).

2. Foot orthesis according to claim 1, characterised in that the plastics material shell (2) is designed to bridge the Chopart's joint by approximately 1 cm distally.

3. Foot orthesis according to claim 1 or 2, characterised by a plastics material shell (2) manufactured using injection moulding technology.

4. Foot orthesis according to claim 3, characterised in that the plastics material shell (2) is made of fibre-reinforced thermoplastics material.

5. Foot orthesis according to one of the preceding claims, characterised in that the edges of the plastics material shell (2) are thickened in a bead-like manner and rounded towards the outside.

6. Foot orthesis according to one of the preceding claims, characterised in that the plastics material shell (2) is at least semi-rigid in the heel region (4).

7. Foot orthesis according to one of the preceding claims, characterised in that the closing mechanism is a touch and close fastening with rollback loop to allow for the variable adjustment of the heel width.

8. Foot orthesis according to one of claims 1 to 6, characterised by a shell section which engages behind the heel region (4), is approximately U-shaped in cross section, is adjustable relative to the plastics material shell (2) in the A-P direction and can be fixed in the desired position to the plastics material shell (2), for example by means of a notch fastening.

9. Foot orthesis according to one of the preceding claims, characterised in that the plastics material shell (2) is equipped with a separate, internal soft wall liner (1) designed to enclose the foot in a sleeve-like manner.

10. Foot orthesis according to claim 9, characterised in that the soft wall liner (1) comprises an entry slot in the region of the dorsum of the foot.

11. Foot orthesis according to claim 9 or 10, characterised in that the soft wall liner (1) has resilient properties and is a closed silicon sleeve.

12. Foot orthesis according to one of the preceding claims, characterised by a fibula wedge (5) fitted laterally in the region of the plastics material shell (2) designed for bridging the dorsum of the foot.

## Revendications

1. Orthèse du pied présentant les particularités suivantes :
- une coque (2) en matière plastique, fermée, uniquement ouverte latéralement dans la zone du talon (4) et pourvue à cet endroit d'un mécanisme de fermeture, la coque se présentant sous la forme d'une orthèse annulaire stable en torsion, adaptée à former un maintien externe dans la région de la cheville;
- la coque (2) en matière plastique présente une zone d'appui (6) de configuration rigide, qui remonte légèrement, et qui est adaptée à venir s'appuyer sur l'extérieur du pied à l'extrémité distale de la fibula (péroné) (7) ;
- la coque (2) en matière plastique est adaptée à recouvrir en direction proximale l'articulation artagalo-scaphoïdienne et calcanéo-cuboïdienne et à l'arthrodéser extérieurement ;
caractérisée en ce que la coque (2) en matière plastique est également adaptée à former un maintien externe de l'interligne articulaire de Chopart,
en ce que ladite zone d'appui (6) de la coque (2) en matière plastique est réalisée sous forme de languette et est d'une configuration particulièrement rigide,
en ce que le bord proximal (8) de la coque (2) en matière plastique se situe, sur le côté intérieur du pied, en-dessous de la malléole interne (9),
et en ce que la coque (2) en matière plastique présente une découpe (10) dans la région plantaire du talon.

2. Orthèse du pied selon la revendication 1, caractérisée en ce que la coque (2) en matière plastique est adaptée à recouvrir l'interligne articulaire de Chopart d'environ 1 cm en direction distale.

3. Orthèse du pied selon la revendication 1 ou 2, caractérisée par une coque (2) en matière plastique fabriquée selon une technique de moulage par injection.

4. Orthèse du pied selon la revendication 3, caractérisée en ce que la coque (2) en matière plastique est réalisée en un thermoplastique renforcé de fibres.

5. Orthèse du pied selon l'une des revendications précédentes, caractérisée en ce que les bords de la coque (2) en matière plastique sont épaissis en forme de bourrelet et arrondis vers l'extérieur.

6. Orthèse du pied selon l'une des revendications précédentes, caractérisée en ce que la coque (2) en matière plastique est d'une configuration au moins semi-rigide dans la zone (4) du talon.

7. Orthèse du pied selon l'une des revendications précédentes, caractérisée en ce que pour le réglage variable de la largeur au talon, le mécanisme de fermeture est constitué par une fermeture auto-agrippante à boucle rabattable.

8. Orthèse du pied selon l'une des revendications 1 à 6, caractérisée par une partie de coque s'engageant derrière la zone (4) du talon et présentant en section transversale une configuration approximativement en forme de U, cette partie de coque pouvant être déplacée dans la direction antéropostérieure par rapport à la coque (2) en matière plastique, et pouvant être fixée, dans la position souhaitée, sur la coque (2) en matière plastique, par exemple par l'intermédiaire d'une fermeture à encliquetage.

9. Orthèse du pied selon l'une des revendications précédentes, caractérisée en ce que la coque (2) en matière plastique est équipée d'un revêtement intérieur à paroi souple (1) séparé, adapté à entourer le pied à la manière d'une manchette.

10. Orthèse du pied selon la revendication 9, caractérisée en ce que le revêtement à paroi souple (1) présente une fente d'entrée, dans la région postérieure du pied.

11. Orthèse du pied selon la revendication 9 ou 10, **caractérisée** en ce que le revêtement à paroi souple (1) présente des propriétés d'élasticité et est une enveloppe fermée en silicone.

12. Orthèse du pied selon l'une des revendications précédentes, caractérisée par un coin de fibula (5), placé latéralement dans la zone de la coque (2) en matière plastique destinée à recouvrir la région postérieure du pied.
